# EUROPEAN PATENT APPLICATION

(11) **EP 4 644 892 A1**
(43) Date of publication of application: **05.11.2025**
(21) Application number: 25165115.4
(22) Date of filing: 20.03.2025
(51) Int. Cl.: G01N 33/00

(54) **GAS SENSOR**

(30) Priority: 03.05.2024 LU 507116
(71) Applicant: FLUSSO LIMITED, Cambridge, Cambridgeshire CB3 0QH (GB)
(72) Inventor: CHIODARELLI, Nicolò, Cambridge, CB3 0QH (GB); UDREA, Florin, Cambridge, CB3 0QH (GB); LEE, Cerdin, Cambridge, CB3 0QH (GB); GARDNER, Ethan Lance, Cambridge, CB3 0QH (GB); ALI, Syed Zeeshan, Cambridge, CB3 0QH (GB)
(74) Representative: Marks & Clerk LLP

(57) **Abstract**

A sensor assembly and a method of manufacturing a sensor assembly. The sensor comprising a gas sensor comprising a gas sensing area, and a package comprising one or more walls, where the walls define a volume accommodating the gas sensor and where one of the walls comprises an aperture defining an inlet for the package. The package comprises a deflecting surface within the volume wherein, in use, the deflecting surface is configured to convey gas molecules towards the gas sensing area.

## Description

### Technical Field

The application relates to gas concentration and composition sensors.

### Background

Thermal conductivity gas sensors are in widespread use in a number of applications due to low cost, small size, and ability to measure a large number of gases and in large range of concentrations.

When used for leak detection of hazardous gasses, a stringent requirement for any gas sensor is having a fast response time. The sensor should detect the leak as soon as possible so that a system can counteract and prevent a potentially destructive outcome, like as an explosion, poisoning or some serious malfunctioning.

Any system's response time, however, is only partly dependent on the type and technology of sensor used within it. A large part of the response time is also determined by the time the gas takes to reach the sensing area of the sensor. Sensors are generally located in a package and/or a casing. It is possible to speed up the transport of gas molecules to a sensor casing or package, for example, by providing a fan, a blower or by exploiting convection and buoyancy. Once the gas molecules reach an aperture in the gas sensor casing or package, though, the time necessary for those molecules to reach the sensing area of the sensor is dictated by gas diffusion.

EP 2 952 886 B1 relates to a method for manufacturing a gas sensor package. US 8,916,408 B2 relates to a leadframe-based premolded package. US 9,708,174 B2 relates to a process for manufacturing a packaged device.

### Summary

The present disclosure is directed to a sensor assembly comprising a thermal conductivity gas sensor and a package enclosing the thermal conductivity gas sensor.

Thermal conductivity gas sensors are responsive to flow, therefore it can be advantageous to prevent gas flow from reaching the sensing area so that the sensor reading may be only determined by the presence and/or concentration of a target gas. This means that gas molecules and, particularly, target gas molecules only reach the sensing area by diffusion.

For a fast and accurate response of the sensor, it is an aim of the present disclosure that the concentration of target gas molecules at the sensing area reaches the value for a representative response (e.g. at least over 50%, or more preferably over 90%, of the concentration of the target gas outside of the package) as fast as possible.

A fast sensor response may depend on the "volume displacement time" i.e. the time necessary for the volume of gas filling the internal volume of a sensing assembly to reach the same composition as the composition of the external environment by gas diffusion or, e.g. at least over 50%, or more preferably over 90%, of the concentration of the target gas outside of the package.

In cases where a sensor is used to detect leaks of hazardous gases, the sensor may be configured to output a sensor response when the concentration of the target gas at the sensing area reaches a detection threshold of the sensor for the target gas. This means that the system response time may be reduced with respect to applications where a precise measurement of the target gas concentration is required..

The sensor assembly of the present disclosure aims at improving the system response time, and/or volume displacement time while avoiding signal interference due to parasitic flow.

Generally, a sensor assembly comprises a sensor, provided with a sensing area, and a package enclosing the sensor. The package may shield the sensor from unwanted contaminations or interferences while ensuring effective interaction of the sensor with the environment for the sensor to function reliably. The package of the sensor assembly according to the disclosure is directed towards improving the system response time and accuracy of the sensor by preferentially directing the diffusing gas molecules, including target gas molecules, towards the sensing area and/or reducing the impact of any potential parasitic gas flow on the sensor response.

The package of the disclosed sensor assembly, in particular the inlet and the internal volume of the package, are configured so that the response time and/or the impact of parasitic flow are minimised.

The diffusion time of a gas from the inlet to the sensing area may depend on the position and dimension of the inlet with respect to the sensing area, on the encumberment of the features inside the package and thus on its internal free volume, all of which may be configured and arranged so as to preferentially direct gas molecules to diffuse towards the sensing area more effectively, so that a preferential diffusion region may be identified inside the package, and/or to reduce the effect of parasitic flow on the accuracy of the sensor reading.

The sensor assembly may be used with gas concentration or composition sensors and leak detectors. The target gas may be H₂. The principles herein disclosed may be applied to any gas composition or concentration sensor, since a fast response time is desirable in any case. The disclosed sensor assembly is of particular relevance for those devices sensing a hazardous gas, such as H₂. Indeed, because any leak of hazardous gas may result in a fatal outcome, a fast response time is an important requirement.

Aspects and preferred features are set out in the accompanying claims.

According to a first aspect of the disclosure, there is provided a sensor assembly comprising:
a gas sensor comprising a gas sensing area;
a package comprising one or more walls, wherein the walls define an volume accommodating the gas sensor and wherein one of the walls comprises an aperture defining an inlet for the package, and wherein the package comprises a deflection surface within the volume, wherein, in use, the deflecting surface is configured to convey gas molecules towards the sensing area.

The deflection surface may encourage preferential diffusion of gas molecules towards the sensing area.

The volume may be an enclosed volume, in that it is surrounded by and enclosed by the walls, with the exception of any inlets, which will allow gas diffusion into the volume. The volume may be referred to as an internal volume.

The deflection surface may extend towards the sensing area of the gas sensor.

Diffusion herein refers to gas diffusion as compared to gas flow (including turbulent and laminar flow).

Generally speaking, diffusion is a phenomenon driven by a concentration gradient. When diffusing molecules of a target gas reach the package inlet, a gradient of concentration is established between the region in close proximity with the inlet and the rest of the package internal volume, including the sensing area.

The system response time, for steady-state measurements, may be determined by the time a concentration change is detected by the sensor, i.e. when the concentration present at the sensing area, reaches a certain percentage of the concentration outside the package. For instance, normally a response time "T50" or "T90" are defined as the time it takes to read a concentration of 50% or 90% of the concentration outside the package, respectively. For hazardous gas and/or leak detection measurements, the system response time may be determined by the time necessary for the concentration of a target gas to reach a predetermined absolute limit or threshold value at the sensing area. Said absolute limit may be the lower detection limit of the sensor for the target gas, or a predefined concentration below a predetermined threshold, for example a lower explosive limit, so that the system may be able to put in place a counter measure or produce a warning signal before a catastrophic event occurs.

For the response of the sensor to be accurate, so that the sensor's signal is truly representative of the concentration and/or presence of the target gas, the sensing area should ideally not be exposed to any gas flow and the gas molecules should only reach the sensing area by diffusion. This is because any gas flow may interfere with the sensor's response.

Providing a large inlet increases the rate of volume displacement of gas into the package by allowing access to the internal volume of the package to a high number of molecules in the unit of time, so as a large amount of molecules are available for the diffusion of the gas to every part of the package. The inlets may have a width of between 100µm and 1mm. For example, the inlets may have a width of at least 500µm. This would thereby decrease sensor response time. However, the package inlet, besides providing an entry point for the gas molecules, also provides a discontinuity in the package that may affect the sensor itself. A large inlet may increase turbulence and/or may allow parasitic flow inside the package, which may also reach the sensing area and, potentially, affect the sensor accuracy. A large inlet also makes it more likely to damage the sensor during handling and assembly (for example, when soldering to a PCB) and will be more susceptible to foreign material ingress.

The inlet may be laterally spaced from the sensing area, so as not to overlap the sensing area. The inlet may be in a non-overlapping relation with the sensing area of the sensor. This means that either the inlet is provided to a wall not facing the sensing area or, if provided to a wall facing the sensing area, a projection of the inlet on the surface of the sensor where the sensing area is located, would not overlap with the sensing area.

This further ensures that the sensing area is not directly exposed to the external environment so that the sensing area may be shielded from any dust, dirt or any foreign matter, which may affect the accuracy of the response of the sensor. An inlet in non-overlapping relation with the sensing area may reduce the effect of any turbulence and/or parasitic flow, on the accuracy of the sensor reading.

The deflecting surface may be configured to reduce an average distance of gas diffused from the inlet to the sensing area.

As used herein, the "main internal volume" or "effective volume" may refer to the portion of the enclosed or internal volume of the package in which the sensor is located.

The one or more walls of the package may be configured to define a preferential diffusion region and a peripheral region within the volume.

As used herein, the expression "preferential diffusion volume" or "preferential diffusion region" may respectively indicate a volume or a region in the main internal volume starting from the inlet, limited, at least in part, by the deflection surface and ending with the sensing area. The preferential diffusion region may include a path defining the physical shortest distance between the inlet and the sensing area of the gas sensor.

It will be understood that the walls defining the internal volume may cooperate with the deflection surface to define the preferential diffusion region.

In preferred embodiments, the preferential diffusion region may provide the shortest diffusion paths from the inlet to the sensing area, and/or a diffusion path, which is not significantly affected by parasitic flow. In some examples, the shortest diffusion path may be shortest physical path between the inlet and the sensing area, which may include physical packaging tolerances and constraints.

The walls may comprise one or more external walls and may additionally comprise one or more internal walls. The deflecting surface may be part of an internal wall of the one or more walls extending from the one or more external walls. The deflection surface may be a surface of an internal wall extending from an external wall into or towards the enclosed volume and towards the sensing area.

The deflecting surface may be monolithic with one of the external walls. In other words, the deflecting surface may be a surface, of an external wall, that faces the interior of the volume.

The deflecting surface may face the inlet. Alternatively or additionally, the deflecting surface may lay adjacent the inlet. The deflection surface may extend towards the inlet and/or towards the sensing area and adjacent the shortest path between the inlet and the sensing area.

The deflection surface may form a non-zero or non-parallel angle relative to the external wall or relative to an external surface of the external wall from which the deflecting surface extends. This deflects gas diffused into the package towards the sensing area and reduces gas from being diffused towards regions of the enclosed volume distant from the sensing area. The deflecting surface may form an obtuse angle facing the inlet, relative to an external surface of a wall from which the deflecting surface extends.

An internal wall comprising the deflecting surface may be oriented as to exclude, at least in part, one or more regions of the main internal volume, which are peripheral with respect to the region between the inlet and the sensing area, from the preferential diffusion region, and/or to shield, at least in part, the sensing area from turbulence and/or parasitic flow.

The package may comprise a bottom wall on which the sensor is located, a top wall opposite the bottom wall and one or more lateral walls.

An internal wall comprising the deflection surface may extend from a top wall and/or from a bottom wall.

An internal wall comprising the deflection surface may extend between two lateral external walls and it may have one or more end portions in contact with one, both or none of the two lateral external walls. The internal wall comprising the deflection surface may provide an obstacle to the diffusion of target gas molecules to peripheral regions of the internal volume of the package.

The internal volume may be formed of two or more distinct or separate volumes. A first volume of the two or more volumes may enclose the preferential diffusion region and a second volume of the two or more distinct volumes may enclose the peripheral region.

An internal wall comprising the deflecting surface may be configured to divide the enclosed volume into two or more distinct volumes or regions. This reduces the effective volume of the package that gas may diffuse into.

The package may further comprise additional internal walls defining at least in part the main internal volume and excluding parts of the enclosed volume from target gas diffusion or hindering the diffusion of target gas molecules to peripheral regions of the enclosed volume.

Portions of the main internal volume where the target gas diffusion is excluded or reduced may be, for example, peripheral empty areas adjacent to external walls or portions of volume occupied by features other than the sensor, e.g. pads, electrical connections and/or other features of the device.

An internal wall comprising the deflection surface may partition the enclosed volume, at least in part, and/or reduce the effective volume of the enclosed volume that gas can diffuse into. Where the internal volume is partitioned by internal walls, an internal main volume and one or more internal peripheral volumes, adjacent to the internal main volume, may be defined. A peripheral internal volume, may not house the sensor but it may house other components of the sensor assembly. An internal peripheral volume may, at least in part, be filled with a suitable material which may be the same or a different material of the walls.

The deflection surface may be monolithic with one of the external walls. The deflecting surface may be integral with an external wall. In these instances, in a direction perpendicular to its external surface, the external wall comprising the deflecting surface may have a first thickness in a region not corresponding to the deflection surface and a second, different thickness in correspondence to the deflection surface.

The external walls may comprise a first region having a first thickness, and a second region having a second thickness. The first and second thicknesses may be thicknesses as measured in a direction perpendicular to the external surface of the external wall. The first thickness may be substantially larger than the second thickness. The first thickness may be at least double the second thickness.

A first external wall of the external walls may have a first portion having the first thickness and a second portion having the second thickness. In other words, one wall of the externals walls may have multiple thicknesses or two or more regions having different thicknesses. The first and second thicknesses may be thicknesses as measured in a direction perpendicular to the external surface of the external wall.

In some embodiments, one or more external walls may have different thicknesses at different locations, in particular they may have larger thicknesses in correspondence of what would otherwise be a peripheral region of the enclosed volume.

In some embodiments, one or more external walls may have different thicknesses at different locations, in particular they may have a smaller thicknesses in an area where an inlet is provided, so that diffusion paths through the inlet are reduced in length and diffusion of gas molecules from outside to inside the package is quicker.

A first external wall of the external walls may have the first thickness and a second external wall of the external walls may have the second thickness. In other words, separate walls of the external walls may have different thicknesses. The first and second thicknesses may be thicknesses as measured in a direction perpendicular to the respective external surfaces. A first wall may have a thickness equal to the first thickness and a second wall may have a thickness equal to the second thickness.

The walls of the package (external and/or internal, when present) may be configured such that the enclosed volume has, at least in part, a similar shape, or profile, to the gas sensor. In other words, at least part of the shape of the internal volume may match the shape of the gas sensor.

In some embodiments, the thickest region of one or more external walls may be adjacent to the sensor so as to match the volume occupied by the sensor and separated from the sensor only by a minimum offset, or clearance, as required for manufacturing and positioning of the sensor. In other words, part of the internal walls may follow part of the perimeter of the sensor.

A distance between a wall of the one or more walls closest to the gas sensor and the gas sensor may be less than 100µm. More preferably, a distance between a wall of the one or more walls and the gas sensor may be less than 50µm.

With respect to a commercial standard package, suitable for MEMS devices, of the same external shape and size, the package of the present disclosure may be described as having reduced internal volume wherein the diffusion of the target gas is restricted or conveyed, through a preferential diffusion volume, to the sensing area.

The one or more walls may comprise a single aperture forming the inlet configured to put the enclosed volume of the sensor assembly in fluidic connection with the external environment with. In other words, the one or more walls may not comprise an outlet, and gas may diffuse into and out of the package only through the single aperture.

The inlet may be circular.

The inlet may be provided in a top external wall of the package. The inlet may be provided in a lateral external wall of the package.

The inlet or aperture may comprise an elongated slot in an external wall, such as a top wall or a side wall of the one or more walls. The inlet may have a length of up to 3mm. The inlet may be formed in a first wall, which may be opposite a second wall of the one or more walls on which the gas sensor is located. The inlet may be formed in a first wall which may be adjacent a second wall of the one or more walls on which the gas sensor is located. The slot may extend in a lateral direction. The slot may have a width in a first direction, which is greater than the width of the gas sensor in the same direction. A large aperture may help enhancing a sensor response time and/or volume displacement time.

The package may comprise two or more inlets.

A first inlet of the two or more inlets may be of the same shape and/or size as a second inlet of the two or more inlets.

Alternatively, first inlet of the two or more inlets may be of different shape and/or size as a second inlet of the two or more inlets.

The sensor assembly may comprise at least two inlets formed in a single wall of the one or more walls. The two or more inlets may be formed in a first wall opposite a second wall of the one or more walls on which the gas sensor is located. The two or more inlets may be formed in a first wall adjacent a second wall of the one or more walls on which the gas sensor is located. The at least two inlets may be laterally spaced from each other.

The at least two inlets may be formed in different walls of the one or more walls.

The one or more walls may comprise a top wall opposing a bottom wall, and one or more side walls extending between the top wall and the bottom wall. The gas sensor may be formed on the bottom wall.

The sensor assembly may further comprise a barrier wall or portion located between the inlet and the gas sensor. The barrier wall may extend from an external or internal wall. The barrier wall may extend from a top wall towards the inlet, or from a region above or below the inlet towards the inlet. The barrier wall may be arranged so that gas molecules diffusing from the inlet to the sensing area have to diffuse around the barrier because a direct diffusion path from an inlet to the sensing area is blocked by the barrier wall. The barrier wall may be particularly useful when the package comprises more than one inlet and/or when an inlet is laterally elongated. In those cases, some parasitic flow may enter the package and the barrier wall may avoid the parasitic flow reaching the sensing area and affecting the signal provided by the sensor.

The sensor assembly may comprise a foreign material collection region between the inlet and the barrier wall where foreign material may collect rather than being pushed around the barrier wall together with the gas molecules.

The barrier wall may direct the gas molecules to diffuse around it towards the sensing area, as a preferred diffusion region. In those embodiments, the preferential region may not comprise the shortest physical diffusion paths with respect to the diffusion region without the barrier wall. On the other hand, such diffusion region may still be defined a preferential diffusion region because it may avoid parasitic flow to affect the sensing area of the sensor.

In some embodiments, the sensor may comprise a substrate or die supporting the sensing area. An inlet may be located above the surface of the substrate or die where the sensing area is provided. A barrier wall may extend from the substrate of the sensor so that, in use, it may deviate the gas molecule diffusing inside the package, so as to direct them to a preferential diffusion region and, in turn toward the sensing area.

In other embodiments, an inlet may be located in a lateral external wall such that the distance between the bottom wall, on which the sensor is located, and the furthest edge of the inlet in a sensor thickness direction, is lower that a thickness of the sensor in the same direction. This may prevent residual flow from reaching the sensing area. In those cases, the lateral wall or walls of the substrate or die facing the inlet may act as a barrier wall.

In a further aspect of the disclosure, there is provided a sensor assembly comprising:
a gas sensor comprising a gas sensing area,
a package comprising one or more walls, wherein the walls define an volume accommodating the gas sensor and wherein one of the walls comprises an aperture defining an inlet for the package, and
   wherein the inlet is laterally spaced from the gas sensing area, and wherein the package further comprises a barrier wall located within the volume, and between the inlet and the gas sensing area

The barrier wall may extend from at least one external or internal wall of the package.

The barrier wall may be configured to define, at least in part, a first region and a second region of the volume, wherein the gas sensor may be located within the first region and wherein the inlet may be located in the second region.

The barrier wall may be configured to reduce gas flow between the inlet and the gas sensing area.

The barrier wall may be arranged so that gas molecules diffusing from the inlet to the sensing area are forced to diffuse around the barrier wall because a direct diffusion path from an inlet to the sensing area is blocked by the barrier wall. The barrier wall may be particularly useful when the package comprises more than one inlet and/or when an inlet is laterally elongated. In those cases, some parasitic flow may enter the package and the barrier wall may avoid the parasitic flow reaching the sensing area and affecting the signal provided by the sensor.

The package may comprise two or more inlets. The two or more inlets may be formed on separate walls of the package.

A first inlet of the two or more inlets may be laterally spaced from a second inlet of the two or more inlets in a first direction. The barrier wall may extend in the first direction.

A first aperture may be located on a lateral wall and a second aperture may be located on a different, e.g. opposite, lateral wall. In other words, a first inlet of the two or more inlets may be formed on a first side wall of the package and a second inlet of the two or more inlets may be formed on a second side wall of the package opposing the first side wall.

Alternatively, a first inlet of the two or more inlets may be formed on a first side wall of the package and a second inlet of the two or more inlets may be formed on a top wall of the package.

The inlet may laterally extend in a first direction, and the barrier wall may extend in the first direction.

This kind of packages may allow fast displacement time but are likely to let residual or parasitic flow into the internal volume. The presence of the barrier wall may shield the sensing area from the residual or parasitic flow and may confine the gas diffusion volume to the volume immediately adjacent the sensor.

The walls of the package may comprise a top wall opposing a bottom wall, and one or more side walls extending between the top wall and the bottom wall, and the gas sensor may be formed on the bottom wall.

The barrier wall may extend from a first side wall of the one or more side walls to a second side wall of the one or more side walls opposite to the first side wall.

The barrier wall may extend from the bottom wall and be in contact with two opposing lateral walls so that gas molecules may diffuse beyond the barrier wall around the top end of the barrier wall. The barrier wall may extend from the top wall and be in contact with two opposing lateral walls so that gas molecules may diffuse beyond the barrier wall around the bottom end of the barrier wall.

The barrier wall may extend between two opposite lateral walls so that gas molecules may diffuse beyond the barrier wall around both top and bottom ends of the barrier wall.

Alternatively, the barrier walls may extend from the top wall to the bottom wall. This allows gas molecules to diffuse beyond the barrier wall around one or both lateral ends of the barrier wall.

The external walls of the package may comprise a first region having a first thickness, and a second region having a second thickness. The first and second thicknesses may be thicknesses as measured in a direction perpendicular to the external surface of the external wall. The first thickness may be substantially larger than the second thickness. The first thickness may be at least double the second thickness.

A first external wall of the external walls may have a first portion having the first thickness and a second portion having the second thickness. In other words, one wall of the externals walls may have multiple thicknesses or two or more regions having different thicknesses. The first and second thicknesses may be thicknesses as measured in a direction perpendicular to the external surface of the external wall.

In some embodiments, one or more external walls may have different thicknesses at different locations, in particular they may have larger thicknesses in correspondence of what would otherwise be a peripheral region of the enclosed volume.

In some embodiments, one or more external walls may have different thicknesses at different locations, in particular they may have a smaller thicknesses in an area where an inlet is provided, so that diffusion paths through the inlet are reduced in length and diffusion of gas molecules from outside to inside the package is quicker.

A first external wall of the external walls may have the first thickness and a second external wall of the external walls may have the second thickness. In other words, separate walls of the external walls may have different thicknesses. The first and second thicknesses may be thicknesses as measured in a direction perpendicular to the respective external surfaces. A first wall may have a thickness equal to the first thickness and a second wall may have a thickness equal to the second thickness.

The walls of the package in the region where the sensor is located may match, at least in part, the shape of the sensor, as described above.

The barrier wall may define a collection region configured to retain any foreign material. In other words, the sensor assembly may comprise a foreign material collection region between the inlet and the barrier wall. Any foreign material may be collected in the foreign material collection region, rather than being pushed around the barrier wall together with the gas molecules.

It will be understood that even though the internal walls or internal sides of the external walls of the packages of the sensor assembly of this disclosure are herein shown as flat surfaces, this is in no way limiting and at least some curved or stepped walls, or walls of any other suitable geometry may be used, when suitable, to improve the effect of the invention.

It will be understood that any internal wall or barrier wall may or may not comprise the same material or materials of the external walls. It will further be understood that the external walls may or may not be made of the same material. In some embodiments, at least a portion of an external wall may comprise a gas permeable membrane, which may function as a first inlet to prevent foreign particles such as dust from entering the enclosed volume of the package. In other implementations, an inlet may be provided with a filter to prevent foreign particles such as dust from entering the enclosed volume of the package, for example a metal mesh filter, a paper filter, a charcoal filter and the like.

The internal walls and/or barrier wall may be made of a polymeric material deposited on the device after fabrication, for instance by spin coating and patterning a thick layer of SU-8 or PDMS.

The package of the sensor assembly may be adapted from commercially available standard MEMS packages, which, normally, have a cuboid shape and an open internal volume. They are readily available and at a low cost. It will be understood, though, that this is not limiting and the package may be different, i.e. not based on standard packages. The package may have any suitable shape, including cuboid, parallelepiped, sphere, spheroid, cylindrical, prism, dome and the like.

The package of the present disclosure may be formed of at least two portions. The walls of the package may comprise a first portion and a second portion. The first portion and the second portion may cooperate to define the volume. The first portion may comprise a package substrate having a cavity in which the gas sensor is located.

In some preferred embodiments, the first portion may comprise a lower wall of the package, on which the sensor is provided. In preferred embodiments, the first portion may comprise a PCB board (known as Chip-on-Board (CoB).

The second portion may comprise a structure that, when located on the first portion, encloses the sensor. The second portion may be a bottomless cuboid-like structure, or a cylinder-like structure or a dome-like structure or any other suitably shaped structure.

The second portion may comprise a top wall of the package. In some embodiments, the second portion is a lid. The lid may be flat or it may be, at least in part convex or concave, with respect to the enclosed volume, to cooperate with the rest of the package walls to accommodate bulky features or components, and/or to further reduce the enclosed volume.

Further portions may be provided, for example, the first portion and/or the second portion may be formed of two or more sub-portions to provide modular packages, which may be adapted to sensors of varied dimensions and/or to different application requirements.

The first portion and the second portion may be fixed to each other in any suitable way known in the art, such as bonded together using a suitable bonding material.

In a further aspect of the disclosure, there is provided a sensor module comprising a casing and the sensor assembly as described above. The casing may house other components and elements of the sensor module, such as a PCB (Printed Circuit Board), a microcontroller and other electronic components. For example, the sensor assembly may be mounted on a PCB, which is then mounted within the casing. The casing or the sensor assembly may have a filter at one or more holes or openings. The casing may have means to make electrical connection between the components inside the casing to the outside world.

The package of the sensor assembly may comprise a single aperture and the casing may comprise two or more apertures. The two or more apertures of the casing may form an inlet and an outlet of the casing. In this way a gas molecule flow may be stablished between the inlet and the outlet of the casing allowing a short volume displacement time for the casing, which, in turn may assure target gas molecules to quickly reach the package of the sensor assembly. The package may be provided with a single inlet to avoid parasitic flow to reach the sensing area or it may be provided with more than one inlet or an elongated inlet in combination with a barrier wall to block any parasitic flow from reaching the sensing area.

The package may comprise two or more apertures and the casing may comprise a single aperture. In other words, gas may flow or diffuse into and out of the casing only through the single aperture. In preferred embodiments, the single aperture of the casing may extend laterally, at least in one direction, to allow a quick change of concentration inside the casing.

According to a further aspect of the disclosure, there is provided a method of manufacturing a sensor assembly, the method comprising:
forming a gas sensor comprising a gas sensing area;
forming a package comprising one or more walls, wherein the walls define a volume accommodating the gas sensor and wherein one of the walls comprises an aperture defining an inlet for the package, and
   wherein the package comprises a deflecting surface within the volume, wherein, in use, the deflecting surface is configured to convey gas molecules towards the sensing area.

According to a further aspect of the disclosure, there is provided a method of manufacturing a sensor assembly, the method comprising:
forming a gas sensor comprising a gas sensing area,
forming a package comprising one or more walls, wherein the walls define a volume accommodating the gas sensor and wherein one of the walls comprises an aperture defining an inlet for the package, and
wherein the inlet is laterally spaced from the gas sensing area, and wherein the package further comprises a barrier wall located within the volume, and between the inlet and the gas sensing area.

The packages may be produced by moulding. Alternatively, the package may be manufactured by additive manufacturing techniques, 3D printing and any other suitable method.

### Brief Description of the Figures

Some embodiments of the disclosure will now be described by way of example only and with reference to the accompanying drawings, in which:
Figure 1(a) shows a top view of a known gas sensor package;
Figure 1(b) shows a perspective view of the internal volume of the package shown in Figure 1(a), as part of a gas sensor assembly;
Figure 1(c) shows simulated diffusion paths through the internal volume shown in Figure 1(b);
Figure 1(d) shows the normalised concentration of a target gas within the inlet (tringles) and at the sensing area of the sensor (circles) for the internal volume shown in Figure 1(b) as a function of time, when the target gas molecules move by diffusion, as shown in Figure 1(c);
Figure 2(a) shows a top view of a gas sensor assembly;
Figure 2(b) shows a top view of a gas sensor assembly;
Figure 2(c) shows the normalised concentration of a target gas within the inlet (tringles) and at the sensing area of the sensor (circles) for the gas sensor assembly of Figure 2(a) as a function of time, when the target gas molecules move by diffusion;
Figure 3(a) shows a top view of a gas sensor package;
Figure 3(b) shows a top view of a gas sensor assembly;
Figure 4 shows a top view of a gas sensor package;
Figure 5 shows a top view of a gas sensor package;
Figures 6(a) to 6(f) show top views of gas sensor assemblies;
Figure 7(a) shows a perspective view of the internal volume of a gas sensor package of a gas sensor assembly;
Figure 7(b) shows simulated diffusion paths through the internal volume shown in Figure 7(a);
Figure 7(c) shows the normalised concentration of a target gas at the sensing area of the sensor, as a function of time, for a gas sensor assembly whose internal volume is shown in Figure 7(a), when the target gas molecules move by diffusion, as shown in Figure 7(b);
Figure 8(a) shows a perspective view of the internal volume of a gas sensor package of a gas sensor assembly;
Figure 8(b) shows simulated diffusion paths through the internal volume shown in Figure 8(a);
Figure 8(c) shows the normalised concentration of a target gas at the sensing area of the sensor as a function of time for a gas sensor assembly whose internal volume is shown in Figure 8(a), when the target gas molecules move by diffusion, as shown in Figure 8(b);
Figures 9(a) to 9(e) show top views of gas sensor assemblies;
Figures 10(a) and 10(b) show cross-sections of gas sensor assemblies;
Figures 11(a) and 11(b) show cross sections of gas sensor assemblies;
Figures 12(a) and 12(b) show cross-sections of gas sensor assemblies;
Figures 13(a) to 13(d) show top views of gas sensor assemblies;
Figures 14(a) to 14(d) show cross-sections of gas sensor assembles with a package formed of at least two portions;
Figures 15(a) and 15(b) show cross-sections of gas sensor modules.

### Detailed Description of the Preferred Embodiments

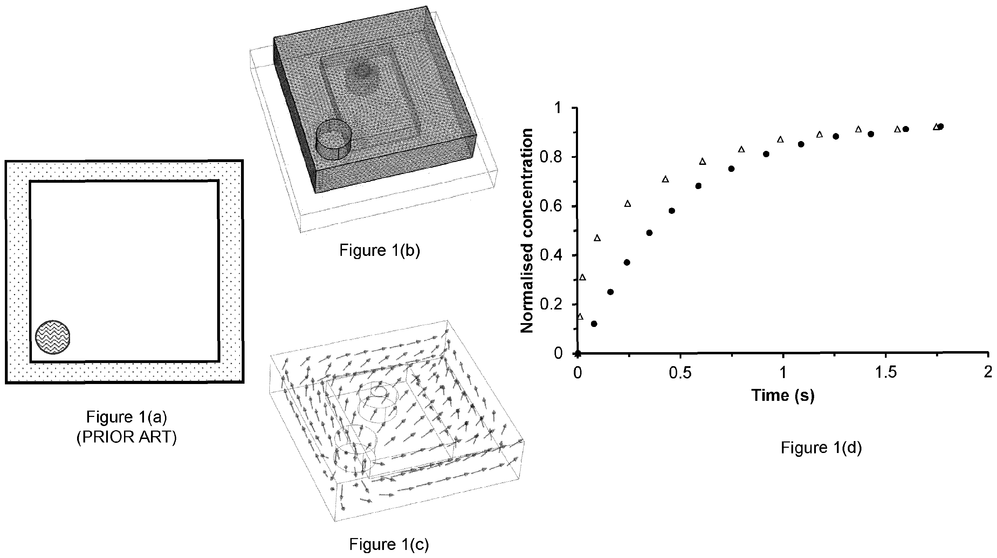

Figure 1(a) shows a top view of a known gas sensor package. The package is generally of a cuboid shape with the side walls shown in Figure 1(a) and a circular inlet.

Figure 1(b) shows a perspective view of the internal volume of a gas sensor assembly comprising a package (not shown, except that for a bottom wall), such as that depicted in Figure 1(a). A gas sensor is located in the internal volume. The gas sensor includes a sensing area formed on a substrate. The package defines an internal volume which gas can enter through an inlet. Figure 1(c) shows simulated diffusion paths through the internal volume depicted in Figure 1(b). This shows that most of the gas molecules entering the package diffuses around the periphery of the volume before reaching the sensitive area of the gas sensor. Figure 1(d) shows the normalised concentration of a target gas at the inlet (triangles) and at the sensing area (circles), as a function of time, for a sensor assembly whose internal volume is shown in Figure 1(b). The sensor response time depends on the time necessary for the normalised concentration of the target gas at the sensing area to reach 90%, in this case, of the concentration of the target gas in the external environment. This is determined by the sum of the time the target gas takes to reach the inlet and the time the target gas takes to reach the sensitive area from the inlet.

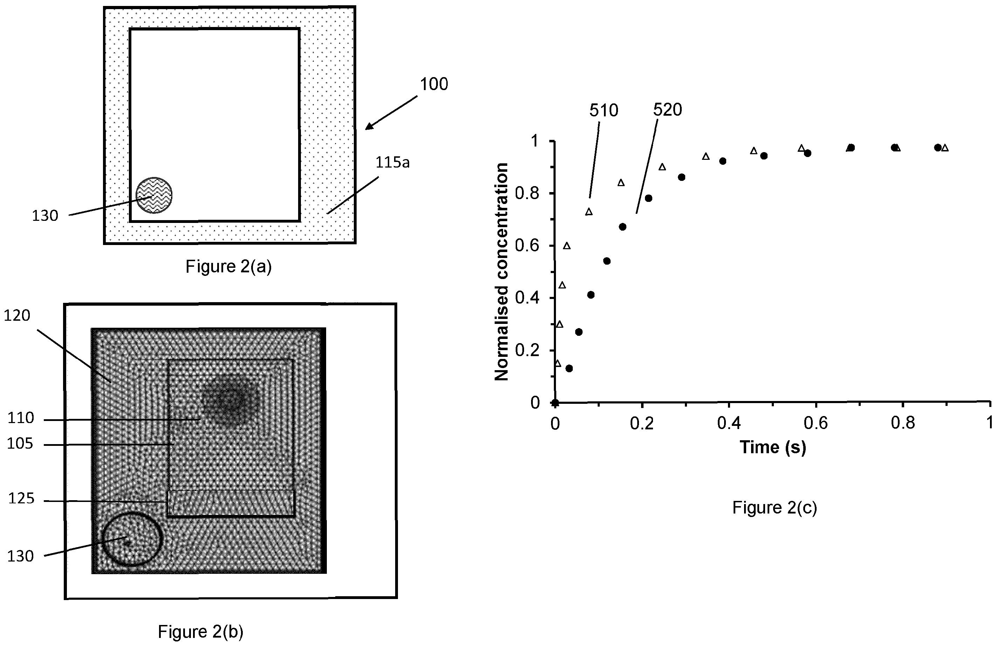

Figure 2(a) shows a top view of the package 100 of a gas sensor assembly according to an embodiment of the disclosure. Figure 2(b) shows a top view of an internal volume 120 of a gas sensor assembly according to an embodiment of the disclosure, having a package (not shown, except for a bottom wall) of reduced internal volume compared to a sensor package such as that shown in Figure 1(a), due to one of the walls, 115a, having a larger thickness compared to the walls shown in Figure 1(a). The gas sensor assembly includes a gas sensor 105 having a gas sensitive area 110. In this example, the package 100 is cuboid shaped although it will be understood that the package may have other shapes. The package 100 includes opposing bottom and top walls (not shown, extending parallel to the plane of the page). The gas sensor 105 is located on the bottom wall of the package. The package includes four side walls 115 extending between the top and bottom walls to define an enclosed volume 120 in which the gas sensor 105 is located. The package 100 of the sensing assembly includes a region 125 where the gas sensor 105 is wire-bonded to the package. An inlet 130 is located in the top wall of the package. The inlet 130 comprises an aperture through the top wall and allows fluid to diffuse from outside the package into the internal volume 120.

As an example, for MEMS sensors, the package may be a cuboid with a base of 2×2mm to 5×5 mm with typical inlets being circular with diameter below 1mm, e.g. 200-500 µm. The thickness of walls of known sensor packages may be in the range 100-200 µm, whereas packages of the present disclosure may have one or more walls having thicknesses of 250-1000 µm.

In the examples shown, the inlet 130 is formed to be laterally spaced from the sensing area 110 and from the bond wires so that the inlet is not located above the sensing area 110 or the bond wires. This reduces interferences of any residual or parasitic flow with the sensing area and damage to the bond wires and sensing area caused by turbulent or bulk flow of the gas to be sensed.

In some examples, the sensor package 100 has a single inlet 130 and may not have an outlet. This reduces bulk flow (including laminar and turbulent flow) into the sensor package 100. This increases the relative influence of gas diffusion on measurements taken by the gas sensor 105, thereby improving the sensitivity of the gas sensor 105 to gas concentration or composition and reducing noise caused by bulk gas flow.

In the example shown in Figure 2(a), one of the side walls, 115a, has a thickness substantially increased relative to the known device shown in Figure 1(a) and relative to the other walls of the device of Figure 2(a), such that the internal volume 120 inside the package is reduced with respect to the internal volume of the example package of Figure 1(a). Figure 2(c) shows results of a simulation of diffusion of a target gas as a normalised concentration 510 of a target gas at the inlet 130 and a normalised concentration 520 of a target gas at the gas sensitive area 110. Compared to the known device of Figures 1(a) to 1(d), it can be seen that increasing the thickness of the wall decreases the time for the gas to diffuse to the gas sensitive area 110. In the example shown in Figure 2(a) and 2(b), the sensor 105 is located between the side wall 115a having an increased thickness and the inlet 130, so that the side wall 115a having an increased thickness reduces the enclosed volume away from a shortest path between the inlet 130 and the gas sensitive area 110. This reduces the average distance that gas from the inlet 130 is diffused before being detected at the gas sensitive area 110. In this example, the side wall 115a with increased thickness has a thickness that is double or more than double the thickness of the other side walls 115b, 115c, 115d. For example, the thickness of the side wall 115a may be in the range 250-1000µm, for example 400µm.

In the examples described herein, the internal walls and walls having increased thickness may reduce the enclosed volume by approximately 10% to 50% compared to known devices, such as that shown in Figures 1(a) to 1(d).

The simulations described herein have been performed using software that models the diffusion of species by solving Fick's laws and related partial differential equations inside a finite volume of air for given dimensions and boundary conditions.

The packages modelled were of 3×3 mm in size, with an inlet, and hosting a silicon die. However, the packages could be larger or smaller. The internal volume was modelled as air at 25 °C and atmospheric pressure. As shown in Figures 1(b), 2(b), 7(a), and 8(a), the internal volume was then divided into a dense mesh, which provided a 3D-framework of fixed points for solving mass transport equations for gas diffusion.

The sensor's response time, T90, was evaluated by simulating the time for the gas concentration directly above the device sensitive area to reach 90% of C₀, the concentration at the inlet. Although the results are normalized, the value used is representative of a typical gas concentration of C₀ = 1% by volume of gas in air. A gas diffusivity D₀ = 10⁻⁵ m²/s was assumed, thus in line with the typical diffusivity of gasses such as water (vapour) and hydrogen (H₂) in standard conditions. The effect of changes in temperature within the -40°C to 125°C range were accounted for by changing the value of diffusivity at each desired temperature.

Pathways through which the gas diffuses inside the package were identified, as shown in Figures 1(c), 7(b), and 8(b). By plotting the concentration vectors over time, it was possible to infer the fastest diffusion path, and to identify those areas possibly presenting a bottleneck for gas diffusion. This is well evidenced in Figure 1(c), which shows that the concentration front proceeds first through the inlet, but then extends along the periphery of the die before finally creeping over the die and reaching the sensitive area.

Figure 2(c) shows that an amelioration greater than 200% (from 1.05s to 0.3s) in T90 is achieved by thickening the side wall 115a of the package 100. The improvement arises from the fact that the gas is forced to proceed straight away toward the gas sensitive area 110 of the device rather than spreading out also toward the right side of the device. In other words, the bottom right diffusion path shown in Figure 1(c) is blocked.

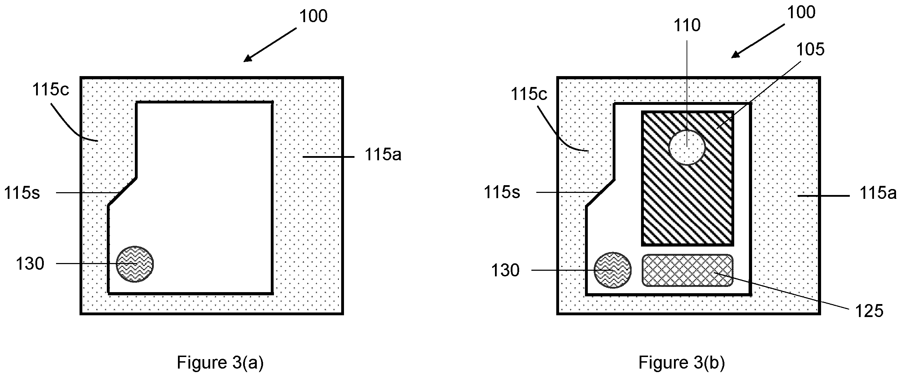

Figure 3(a) shows a top view of the walls of a gas sensor package 100 according to an embodiment of the disclosure and Figure 3(b) shows a top view of an example gas sensor assembly having the package shown in Figure 3(a). The gas sensor package is similar to that shown in Figure 2(a) and therefore carries similar reference numerals. In this example, the side walls 115 have different thickness. If we define thickness t as a first thickness, at least a portion of a side wall has a thickness tₓ higher than t. In the example of Figures 3(a) and (b), side wall 115a has a thickness higher than t, throughout. The side wall 115c has a region having thickness t and a region having a thickness higher than t. The portion of the side wall 115c having thickness higher than thickness t is distant from the inlet 130. In the example shown, the inlet 130 is between the portion of the side wall 115c without an increased thickness and the gas sensitive area 110. It will be understood that, whilst the example of Figure 3(a) shows side wall 115c having a region having thickness t and a region having a thickness higher than t, other side walls may alternatively or additionally have a region having thickness t and a region having a thickness higher than t. In general, the side walls may have at least a first portion having a first thickness, and a second portion having a second thickness, where the second thickness is substantially greater than the first thickness.

The portion of side walls 115 having thickness higher than t reduces the enclosed volume away from a shortest path between the inlet 130 and the gas sensitive area 110 with respect to the case of a package with all the side walls of thickness t. This reduces the average distance that gas from the inlet 130 is diffused before being detected at the gas sensitive area 110. In this example, the portion of the side wall 115c with increased thickness has a thickness that is double the thickness of the regions without increased thickness. In some examples, the portion of the side wall 115c with increased thickness has a thickness that is more than double the thickness of the regions without increased thickness. The thickness of side wall 115c is tapered so that the change in thickness is not sudden. This avoids blind spots and corners that may produce turbulence between the inlet and the sensing area, and provides a surface, 115s, which is a deflecting surface extending towards the sensing area so as to convey the target gas molecules entering the package preferentially towards the sensing area, rather than towards the peripheral region at the corner between walls 115c and 115b. In this embodiment the deflection surface 115s is monolithic with the external wall 115c.

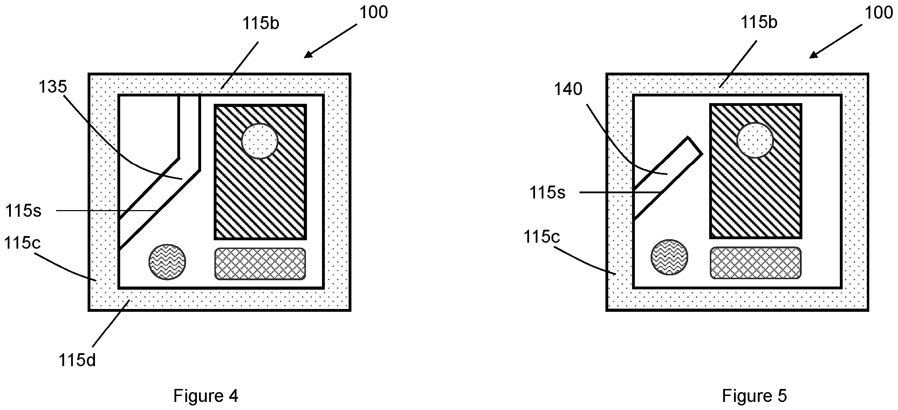

Figure 4 and 5 shows a top view of gas sensor assemblies according to further embodiments of the disclosure. In the example of Figure 4, an internal wall 135 is located inside the package. The internal wall 135 divides the internal volume inside the package into a main internal volume, containing the gas sensor, and a peripheral internal volume, thereby reducing the volume containing a gas sensor. This can be used to reduce the enclosed volume away from a shortest path between the inlet 130 and the gas sensitive area 110, thereby reducing the average distance that gas from the inlet 130 is diffused before being detected at the gas sensitive area 110. The internal wall 135 may be in contact with the top wall and the bottom wall of the package so as to completely exclude the peripheral internal volume from the diffusion of gas entering the package. The internal wall 135 may be in contact with only one or none of the top and bottom walls of the package so as to provide an obstacle to gas diffusion to the peripheral internal volume so that the number of molecules diffusing towards the peripheral internal volume in the unit of time is lower with respect to the number of molecules diffusing in the main internal volume in the unit of time. The deflection surface 115s in this case is comprised in the internal wall 135 and conveys diffusing gas molecules preferentially towards the sensing area.

In this example, the internal wall extends between two adjacent side walls 115b, 115c, although it may extend between two opposite side walls or any other side walls.

In the example gas sensor assembly of Figure 5, one end of an internal wall 140 extends from one side wall 115c only, and another end of the internal wall 140 is not joined to any side wall. The internal wall 140, specifically the deflection surface 115s, which is part of the internal wall 140, deflects any gas, to block or reduce gas diffusion to a region between the internal wall 140 and the side walls 115c, 115b. This increases diffusion along or close to the shortest path between the inlet 130 and the gas sensitive area 110, thereby reducing the average distance that gas from the inlet 130 is diffused before being detected at the gas sensitive area 110. The internal wall 140 may preferably be in contact with both the top and bottom walls of the package so that the only diffusion route towards the peripheral internal volume is at the side of the internal wall. The internal wall 140 may be in contact with only one or neither of the top and bottom walls of the package so as to still provide an obstacle to most diffusion routes towards the peripheral internal volume while still providing some additional diffusion paths between the internal wall 140 and the top and/or bottom walls of the package.

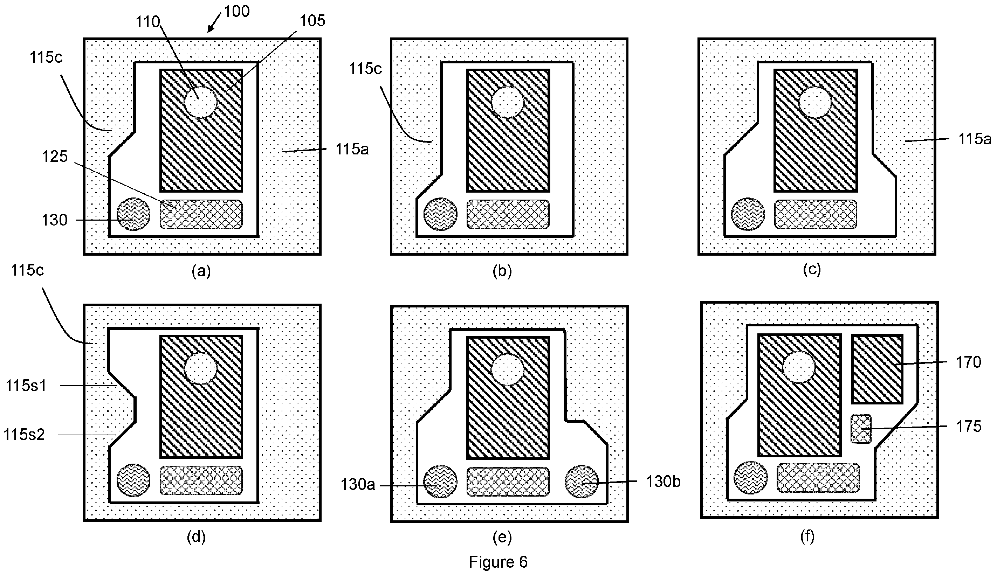

Figures 6(a) to 6(f) show different embodiments of the package of the sensor assembly according to the present disclosure.

Figure 6(a) shows a gas sensor package similar to that show in Figure 3(a).

Figure 6(b) shows a gas sensor package similar to that shown in Figure 6(a), however the thicker portion of the side wall 115 extends over a greater length of the side wall 115c and the deflection surface 115s is adjacent to the inlet 130. This further reduces the enclosed volume away from a shortest path between the inlet 130 and the gas sensitive area 110, thereby reducing the average distance that gas from the inlet 130 is diffused before being detected at the gas sensitive area 110.

Figure 6(c) shows a gas sensor package similar to that shown in Figure 6(a), however two opposing side walls 115a, 115c have regions of different thicknesses, rather than one of the side walls being a constant thickness.

Figure 6(d) shows a gas sensor package similar to that shown in Figure 6(a), however, the side wall 115c has a central region have an increased thickness between two regions without increased thickness. The two deflection surfaces 115s1 and 115s2 deflect gas, to block or reduce gas diffusion to a region distant behind the central region and distant from the inlet. This increases diffusion along or close to the shortest path between the inlet 130 and the gas sensitive area 110, thereby reducing the average distance that gas from the inlet 130 is diffused before being detected at the gas sensitive area 110.

Figure 6(e) shows a gas sensor package similar to that shown in Figure 6(a), however in this example, two inlets 130a, 130b are located in the package. This allows for increased diffusion into the package, though can also allow residual gas flow into the package. The side wall 115c adjacent to the second inlet 130b has a region having increased thickness. The portion of the side wall 115c having an increased thickness is distant from the inlet 130a, where the inlet 130a is between the portion of the side wall 115a without an increased thickness and the gas sensitive area 110. The portion of side wall 115c having an increased thickness reduces the enclosed volume away from a shortest path between the second inlet 130b and the gas sensitive area 110. This reduces the average distance that gas from the inlet 130b is diffused before being detected at the gas sensitive area 110.

Figure 6(f) shows a gas sensor package similar to that shown in Figure 6(a). In this example, the gas sensor package includes more than one die, for example, a system constituted of a sensor 105 and an ASIC readout chip 170. A second electrical connection region 175 is formed for the ASIC readout chip. The additional die, in this example the ASIC readout chip 170, reduces the internal volume thereby reducing the average diffusion distance to the gas sensitive area 110 and reducing sensor response times.

In the examples shown, the side walls are configured to reduce the enclosed volume within the package.

As it is apparent, the walls of the packages shown in Figures 6(a) to 6(f)or, in other words, the internal volume that they define, match, at least in part the shape of the sensor and, where present, other components of the sensing assembly. In other words, the side walls are configured such that a distance between the side walls and the components within the package is reduced. In some examples, the distance between the gas sensor 105 and the side walls may be less than 100µm, or less than 50µm.

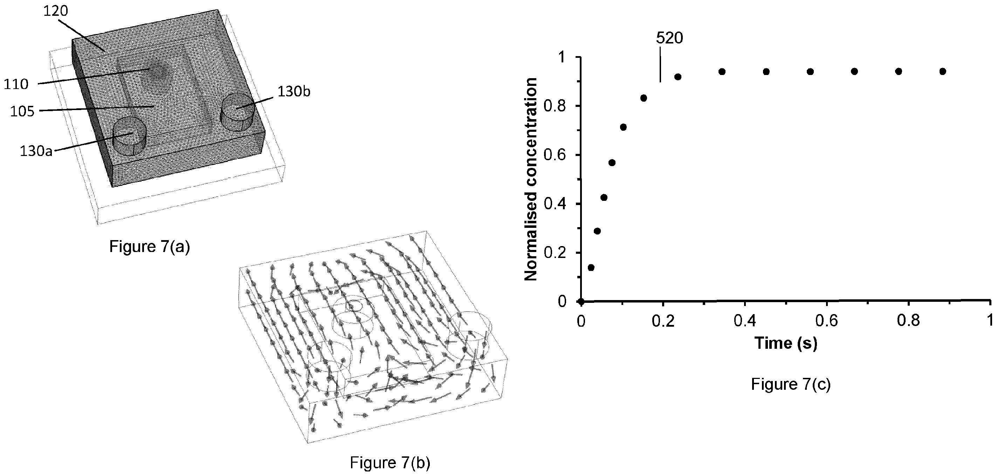

Figure 7(a) shows a perspective view of the internal volume of a gas sensor assembly. The internal volume 120 is similar to that shown in Figure 1(b), and therefore similar reference numerals are provided. In this case, the package includes two inlets 130a and 130b. The package (not shown, except for a bottom wall) defines an internal volume through which gas can enter from both inlets. A barrier wall may be present opposite the sensing area and adjacent to the two inlets, although it will be understood that other examples may not include a barrier wall. Figure 7(b) shows simulated diffusion paths through the internal volume 120 shown in Figure 7(a). Most of the gas diffuses around the periphery of the volume before reaching the sensitive area of the gas sensor. During operation, the presence of the two inlets or apertures may allow some residual flow inside the internal volume of the package, and thus a barrier wall may be located between the two inlets and the sensing area in order to reduce any residual flow reaching the sensing area. Figure 7(c) shows that T90 (0.19s) is reduced with respect to that reported in Figure 1(d) thanks to the presence of the second inlet.

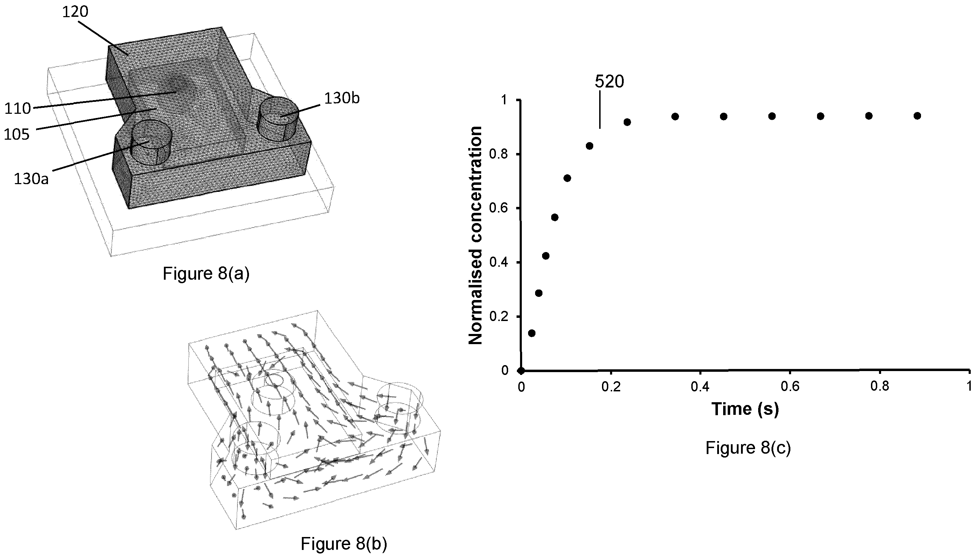

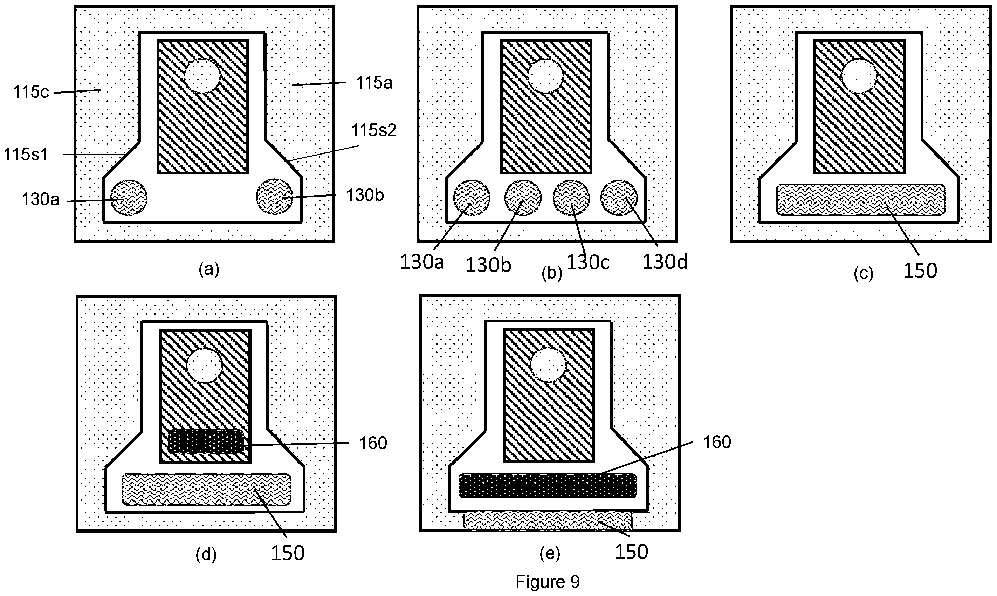

Figure 8(a) shows a perspective view of the internal volume of a gas sensor assembly according to an embodiment of the disclosure. The gas sensor assembly is similar to those shown in Figure 2 and 6, and therefore similar reference numerals are provide. The internal volume 120 shown in Figure 8(a) may be defined by the package of Figure 9(a) where two inlets 130a, 130b are provided through the top wall of the package, thereby allowing for increased diffusion into the internal volume of the package. Side walls 115a, 115c adjacent to the first inlet 130a and the second inlet 130b, respectively, both have a region having increased thickness. The portions of the side walls 115a, 115c having an increased thickness are distant from the inlets 130a, 130b, whereas the inlets 130a, 130b are between the portion of the side walls 115a, 115c without an increased thickness and the gas sensitive area 110. The portions of the side walls 115a, 115c having an increased thickness reduce the enclosed volume away from a shortest path between the inlets 130a, 130b and the gas sensitive area 110. This reduces the average distance that gas from the inlets 130a, 130b is diffused before being detected at the gas sensitive area 110. Deflection surfaces 115s1 and 115s2 convey gas molecules diffusing inside the package preferentially towards the gas sensitive area 110. A (optional) barrier wall may reduce any parasitic flow, if any, which may enter the internal volume, reaching the gas sensitive area 110 of the gas sensor and having a detrimental effect on the sensor accuracy.

Figure 8(b) shows diffusion paths through the internal volume shown in Figure 8(a), and Figure 8(c) shows the normalised concentration at the sensing area as a function of time for the internal volume shown in Figure 8(a). From this, it can be seen that having the portions of the side walls with increased thickness, the deflection surfaces and two inlets instead of a single inlet further decreases T90 (0.155 s).

Including two inlets may have a similar effect as increasing the area of an inlet in examples having a single inlet. Enlarging the inlet, or doubling the inlet's number, leads to a shorter response time.

Figures 9(a) to 9(e) show various embodiments of the gas sensor assembly according to the present disclosure, having more than one inlet or an inlet which extends laterally.

Figure 9(a) shows a gas sensor package similar to that show in Figure 8(a). In this example, two inlets 130a, 130b are located in the package. This allows for increased diffusion into the package, though can also allow residual gas flow into the package. Side walls 115a, 115c adjacent to the first inlet 130a and the second inlet 130b, respectively, both have a region having increased thickness. The portions of the side walls 115a, 115c having an increased thickness are distant from the inlets 130a, 130b, whereas the inlets 130a, 130b are between the portion of the side walls 115a, 115c without an increased thickness and the gas sensitive area 110. The portions of the side walls 115a, 115c having an increased thickness reduce the enclosed volume away from a shortest path between the inlets 130a, 130b and the gas sensitive area 110. This reduces the average distance that gas from the inlets 130a, 130b is diffused before being detected at the gas sensitive area 110.

Figure 9(b) shows a gas sensor package similar to that show in Figure 9(a). In this example, four inlets or apertures are provided. This may further increases gas diffusion into the sensor assembly.

Figure 9(c) shows a gas sensor package similar to that show in Figure 9(a), however in this example, the inlets are replaced with an elongated slot inlet 150 through a top wall of the package. This allows access to the main internal volume to a higher amount of gas molecules per unit of time.

Figure 9(d) shows a gas sensor package similar to that shown in Figure 9(c). In this example, the sensor assembly includes a barrier wall 160 between the inlet 150 and the sensing area 110. The barrier wall 160 may extend from a top wall of the package towards the gas sensor 105. The barrier wall 160 reduces parasitic gas flow from the elongated inlet 150 reaching the gas sensitive area 110 whilst still allowing gas diffusion towards the gas sensitive area 110. In some examples, the barrier wall 160 may extend from the substrate of the sensor towards a top wall so that, in use, it may deviate the gas molecule diffusing inside the package. The barrier wall 160 is located laterally between the inlet 150 and the gas sensitive area 110.

Figure 9(e) shows a gas sensor package similar to the one shown in Figure 9(d). In this further example, the package is provided with an elongated inlet located on a side wall rather than on a top wall. A barrier wall 160 may extend from a top wall of the package towards the bottom wall and/or the sensor. The barrier wall 160 is located laterally between the inlet 150 and the sensing area 110. The barrier wall 160 reduces parasitic gas flow from the elongated inlet 150 reaching the gas sensitive area 110 whilst still allowing gas diffusion towards the gas sensitive area 110.

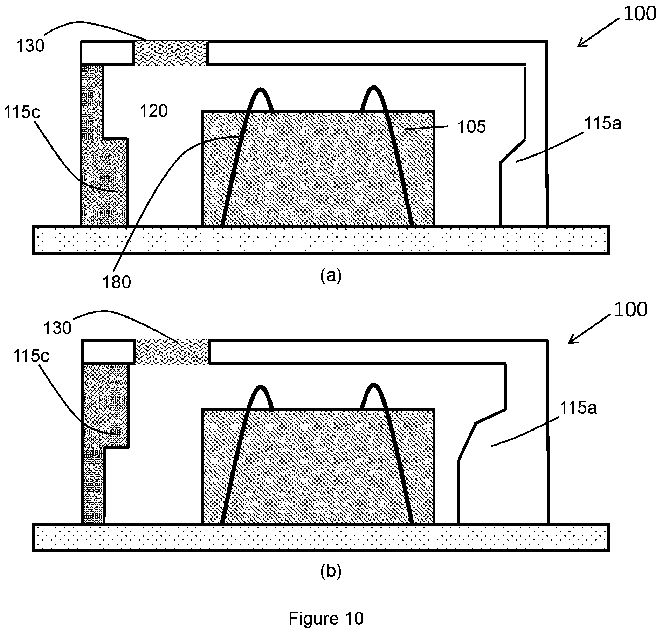

Figure 10(a) shows a cross-section of a gas sensor assembly having a package 100, such as those shown in Figures 2 to 9. The sensor package may be similar to those shown in Figures 2 to 9 and therefore carries similar reference numerals. In this example, the two side walls shown 115a, 115c, have an increased thickness towards a lower portion of the side walls. This reduces the enclosed volume within the package 100, thereby reducing average diffusion distance and improving sensor reaction times. The sensor 105 is provided with electrical connections 180.

Figure 10(b) shows a cross-section of a gas sensor assembly having a package such as those shown in Figures 2 to 9. The sensor package may be similar to those shown in Figures 2 to 9 and therefore carries similar reference numerals. In this example, one side wall 115c has an increased thickness towards an upper portion of the side wall and an opposite side wall 115a has an increased thickness towards a lower portion of the side wall. This reduces the enclosed volume within the package 100, thereby reducing average diffusion distance and improving sensor response times. In general, the side walls may have a thickness that varies vertically as well as horizontally.

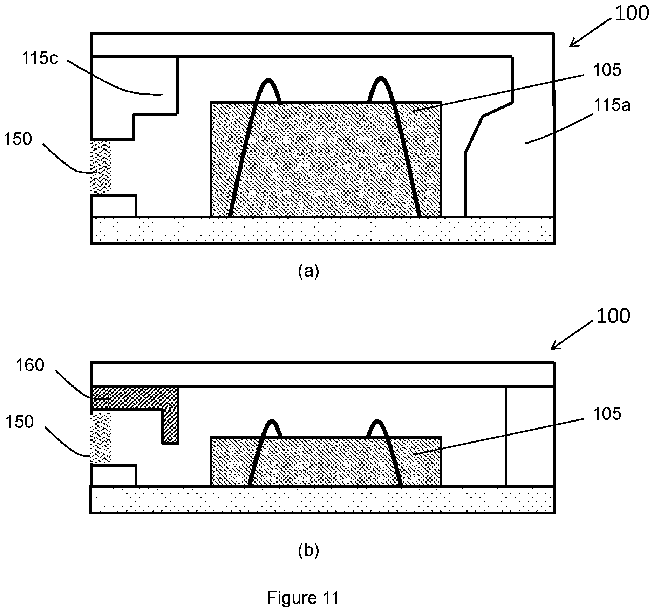

Figure 11(a) shows a cross-section of a gas sensor assembly. The sensor package may be similar to those shown in Figures 2 to 10 and therefore carries similar reference numerals. In this example, the inlet 150 is formed in a side wall 115c of the package. In this example, the inlet 150 is located lower than the upper surface of the sensor 105 and the gas sensitive area 110, so that the gas sensitive area is above the inlet 150. In this example, a lateral wall or walls of the gas sensor 105 facing the inlet 150 may also act as a barrier wall. This reduces parasitic gas flow over the sensitive area 110. The inlet 150 is formed as an aperture through the side wall 115c of the package. The side wall 115c having the inlet has a region having an increased thickness above and distal from the inlet 150. The side wall 115a opposite and distal from the inlet 150 has a region of increased thickness on a lower portion of the side wall.

Figure 11(b) shows a cross-section of a gas sensor assembly with an alternative gas sensor package. The sensor package may be similar to those shown in Figures 2 to 11(a) and therefore carries similar reference numerals. In this example, the inlet 150 is formed in a side wall 115c of the package, and a barrier wall 160 is formed between the inlet 150 and the sensing area. The inlet 150 may be laterally elongated as shown in Figure 9(e). The barrier wall 160 extends from a top wall of the package towards the bottom wall and is located, at least in part, opposite the inlet, so as to prevent parasitic flow from reaching the sensitive area of the gas sensor 105.

Laminar or turbulent parasitic or residual flow reaching the sensitive area is detrimental and interferes with the sensor's measurements. The occurrence of residual flow entering the package may be especially likely in embodiments having more than one inlet, as an aperture may function as inlet and another as outlet and/or in embodiments having an elongated slot inlet, especially when a bulk flow is present outside the package, for example in order to reduce the time necessary for target gas molecule to reach the sensor package.

The barrier wall 160 extending towards the inside of the package, between the inlet and the sensing area, mitigates against parasitic flow from reaching the gas sensitive area 110. This barrier wall 160 will direct the gas molecules to diffuse around it to avoid spreading the parasitic flow to the sensing area and the one or more deflecting wall, for example as shown in Figure 9(e), will convey the gas molecules towards the sensing area, as a preferred diffusion region.

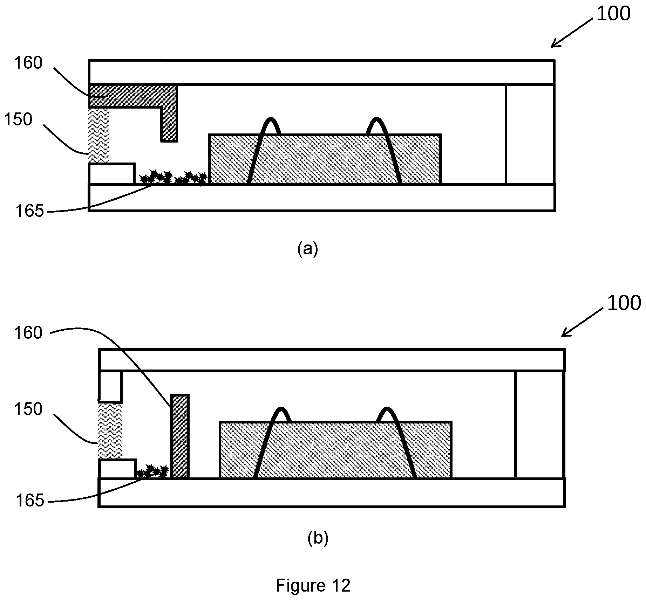

The barrier wall 160 may alternatively extend from the bottom wall, as shown in Figure 12(b). The barrier wall 160 may help with avoiding that foreign particles and dust reach the sensing area. The particles and dust may collect at the base or below the barrier wall 160 rather than travelling around the barrier wall with the gas molecules, as shown in Figure 12(a). In these cases, the package may be provided with a foreign material collection portion 165, as shown in Figures 12(a) and 12(b). As discussed, having the one or more inlets in non-overlapping relation with the sensing area also helps with preventing particles and dust from reaching the sensing area. These features may also be used in combination.

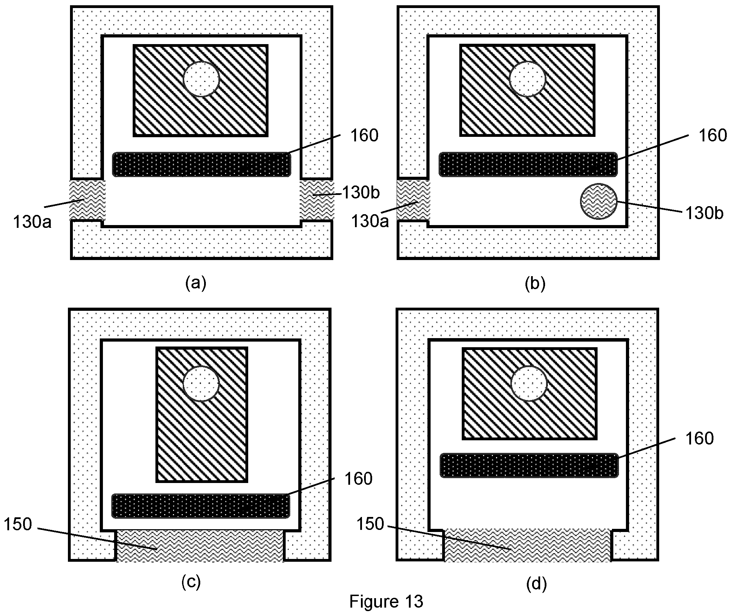

Figures 13(a) to 13(d) show top views of examples of sensor assembles according to embodiments of the present disclosure.

The package shown in Figure 13(a) has two apertures 130a, 130b on opposite side walls and a barrier 160 between the region where the two apertures are provided and the sensing area 110. The barrier wall 160 may divide the internal volume into a first region where the sensor 105 is located and a second region where apertures 130a and 130b are located. A gas flow may be established between the two apertures 130a and 130b. The barrier wall 160 extends in the same general direction of the gas flow, but this is not limiting and the barrier wall 160 may be at an angle with respect to the flow direction. Gas molecules may diffuse to the sensing area around the barrier 160 without the parasitic flow significantly affecting the sensing area. The package of Figure 13(b) is similar to the one shown in Figure 13(a) but, in this case, one aperture 130a is provided on a side wall and another aperture 130b on a top wall.

Figures 13(c) and 13(d) show packages where, instead of two apertures, only one aperture 150 is provided in a side wall, extending in a lateral direction. A barrier 160 is located opposite the inlet 150 to shield the sensing area from any parasitic flow. The barrier 160 is located between the inlet 150 and the sensing area 110. As shown in Figure 13(d), the barrier 160 may be arranged so that the volume where the sensor 105 is located is reduced compared to Figure 13(c).

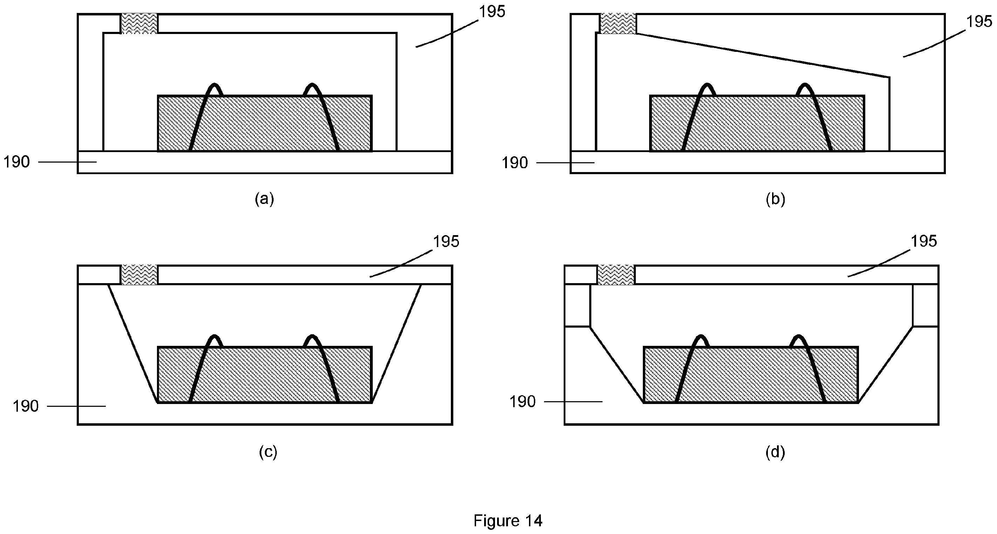

Figures 14(a) to 14(d) show cross-sections of further examples of sensor packages according to the present disclosure. In each of these embodiments, the package may be formed of two or more portions. The sensor package may be similar to those shown in Figures 2 to 11 and therefore carries similar reference numerals.

Figures 14(a) and 14(b) depict packages having a first portion 190, which is a bottom wall, for example a substrate or a PCB board. In this example, the sensor 105 is formed on an upper surface of the bottom or lower wall. A second portion 195 of the package encloses the sensor 105 laterally and on the top.

The sensor package shown in Figure 14(a) has one side wall of a greater thickness to the opposite side wall, such as the sensor package shown in Figure 2(a).

The sensor package shown in Figure 14(b) has one side wall of a greater thickness to the opposite side walls, similar to the sensor package shown in Figure 2(a) and Figure 14(a). The sensor package shown in Figure 14(b) has a sloping top wall comprising a deflecting surface. The side walls of a greater thickness of the packages of Figures 14(a) and 14(b) help convey the gas molecules to the sensing area.

Figures 14(c) and 14(d) depict packages having a first portion 190, which is a package substrate provided with a cavity on a top surface of the substrate. The sensor 105 is located on the substrate, within the cavity. In the examples shown, each of the walls of the substrate, formed by the cavity, have varied thickness so as to help conveying the gas molecules to the sensing area of the sensor 105. A second portion 195 of the package encloses the sensor on the top. In some examples, the second portion 195 may have walls of different thickness. In Figure 14(d) the first portion is formed of two or more sub-portions; this provides a modular package, which may be adapted to sensors of varied dimensions and/or to different application requirements

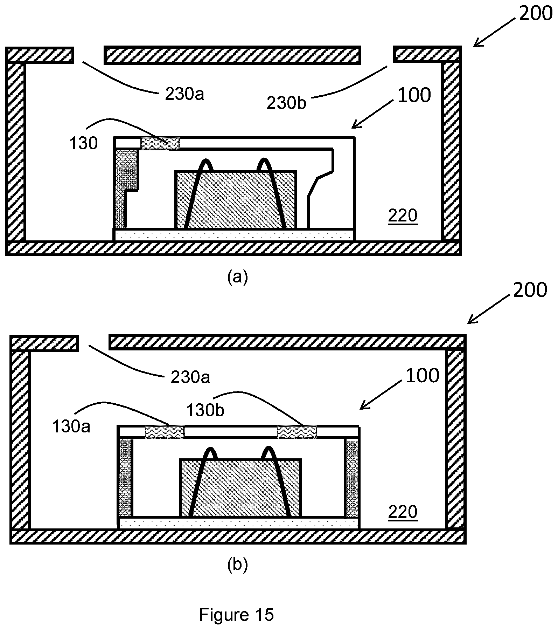

Figure 15(a) shows a cross-section of a gas sensor module. The gas sensor module includes a gas sensor assembly, comprising a package 100, and a casing 200 where the sensor assembly is located. In this example, the gas sensor package 100 is similar to that shown in Figure 10(a) and therefore carries similar reference numerals, though it will be understood that any other gas sensor packages herein described could be implemented inside the casing 200. In this example, the casing 200 has a cuboid shape although it will be understood that the casing can be other shapes. The casing 200 includes opposing bottom and top walls and four side walls extending between the top and bottom walls to define an enclosed volume 220 in which the gas sensor package 100 is located. The gas sensor package 100 is provided on a bottom wall of the casing and is enclosed by the casing 200. In this example, the gas sensor package has a single inlet 130 and the casing 200 has two apertures 230a, 230b located on a top wall of the casing 200. In this way, gas molecules to be sensed can flow quickly into the casing 200 (with one aperture acting as an inlet 230a, and the other as an outlet 230b), but has to diffuse into the sensor package 100 - thereby reducing parasitic flow from affecting the sensor 105. This allows the casing 200 to be filled with gas quickly, thereby improving the sensor response time. In this example, the casing 200 has two apertures or holes, but can also have more than two holes. The apertures may be provided to the same or to different walls of the casing 200.

Figure 15(b) shows a cross-section of a further example of a gas sensor module. The sensor assembly may be similar to that shown in Figure 15(a) and therefore carries similar reference numerals. In this example, the gas sensor package 100 has two apertures 130a, 130b and the casing has a single inlet 230. In this example, the gas to be sensed has to diffuse into the casing 200, and then diffuses into the package 100 as there is no bulk flow within the casing 200. In this case, it takes longer to fill the casing 200, but once the casing 200 is filled with gas, the diffusion into the sensor package 100 is faster due to the presence of more than one aperture in the package 100. It should be noted that there can be more than two apertures within the sensor package 100.

The skilled person will understand that, in the preceding description and appended claims, positional terms such as 'above', 'overlap', 'under', 'lateral', etc. are made with reference to conceptual illustrations of a device, such as those showing standard cross-sectional perspectives and those shown in the appended drawings. These terms are used for ease of reference but are not intended to be of limiting nature. These terms are therefore to be understood as referring to a device when in an orientation as shown in the accompanying drawings.

Although the disclosure has been described in terms of preferred embodiments as set forth above, it should be understood that these embodiments are illustrative only and that the claims are not limited to those embodiments. Those skilled in the art will be able to make modifications and alternatives in view of the disclosure, which are contemplated as falling within the scope of the appended claims. Each feature disclosed or illustrated in the present specification may be incorporated in the disclosure, whether alone or in any appropriate combination with any other feature disclosed or illustrated herein.

### Reference Numerals

- 100: Gas sensing package
- 105: Gas sensor
- 110: Gas sensitive area
- 115: Package side walls
- 120: Enclosed volume
- 125: Wire bonding region
- 130: Package inlet
- 135: Internal wall
- 140: Internal wall
- 150: Inlet
- 160: Internal barrier wall
- 165: Foreign material collection portion
- 170: ASIC readout chip
- 175: Electrical connection region
- 180: Electrical connections
- 190: First portion
- 195: Second portion
- 200: Casing
- 220: Enclosed volume
- 230: Casing aperture

## Claims

1. A sensor assembly comprising:
a gas sensor comprising a gas sensing area;
a package comprising one or more walls, wherein the walls define a volume accommodating the gas sensor and wherein one of the walls comprises an aperture defining an inlet for the package, and
wherein the package comprises a deflecting surface within the volume wherein, in use, the deflecting surface is configured to convey gas molecules towards the gas sensing area.

2. The sensor assembly according to claim 1, wherein the inlet is laterally spaced from the gas sensing area, and wherein, in use, the deflecting surface is configured to reduce an average distance of gas when diffusing from the inlet to the sensing area.

3. The sensor assembly according to claim 1 or 2, wherein the one or more walls of the package are configured to define a preferential diffusion region and a peripheral region within the volume; and
optionally wherein the volume is formed of two or more distinct volumes, and wherein a first volume of the two or more distinct volumes encloses the preferential diffusion region and wherein a second volume of the two or more distinct volumes encloses the peripheral region.

4. The sensor assembly according to claim 1, 2, or 3, wherein the walls comprise one or more external walls of the sensor package and an internal wall extending from an external wall, and wherein the deflecting surface is part of the internal wall; and/or
wherein the walls comprise one or more external walls of the sensor package, and wherein the deflecting surface is monolithic with one of the external walls.

5. The sensor assembly according to claim 4, wherein the deflecting surface forms an obtuse angle facing the inlet, relative to an external wall from which the deflecting surface extends.

6. The sensor assembly according to claim 4 or 5, wherein the external walls comprise a first region having a first thickness, and a second region having a second thickness.

7. The sensor assembly according to claim 6, wherein the first thickness is at least double the second thickness; and/or
wherein a first external wall of the external walls has the first thickness and wherein a second external wall of the external walls has the second thickness; and/or
wherein a first external wall of the external walls has a first portion having the first thickness and a second portion having the second thickness.

8. The sensor assembly according to any preceding claim, wherein the shape of the volume matches, at least in part, a shape of the gas sensor; and
optionally wherein a distance between a wall of the one or more walls and the gas sensor is less than 100µm, more preferably wherein a distance between a wall of the one or more walls and the gas sensor is less than 50um.

9. The sensor assembly according to any preceding claim, wherein the aperture comprises a slot in a first wall of the one or more walls and wherein the slot extends in a lateral direction.

10. The sensor assembly according to any preceding claim, wherein the sensor assembly comprises at least two inlets formed in a first wall of the one or more walls, wherein the two inlets are laterally spaced from each other; or
wherein the one or more walls comprise a single aperture forming the inlet.

11. The sensor assembly according to any preceding claim, wherein the walls comprise a top wall opposing a bottom wall, and one or more side walls extending between the top wall and the bottom wall, and
wherein the gas sensor is located on the bottom wall, and
wherein the inlet is formed on one of the side walls.

12. The sensor assembly according to claim 11, wherein a distance of a top surface of the gas sensor from the bottom wall of the package is higher than the distance of a top edge of the inlet from the bottom wall of the package; or
further comprising a barrier wall located between the inlet and the gas sensing area.

13. The sensor assembly according to any preceding claim, wherein the package comprises a first portion and a second portion, and wherein the first portion and the second portion cooperate to define the volume, and wherein the first portion comprises a substrate having a cavity in which the gas sensor is located.

14. A sensor module comprising the sensor assembly according to any preceding claim and a casing, wherein the sensor assembly is located within the casing; and
optionally wherein the casing comprises two or more apertures; and
optionally wherein the package comprises a single aperture; or
wherein the package comprises two or more apertures and wherein the casing comprises a single aperture.

15. A method of manufacturing a sensor assembly, the method comprising:
forming a gas sensor comprising a gas sensing area;
forming a package comprising one or more walls, wherein the walls define a volume accommodating the gas sensor and wherein one of the walls comprises an aperture defining an inlet for the package, and wherein the package comprises a deflecting surface within the volume, wherein, in use, the deflecting surface is configured to convey gas molecules towards the gas sensing area.
